# EUROPÄISCHE PATENTSCHRIFT

(11) **EP 3 705 889 B1**
(45) Veröffentlichungstag und Bekanntmachung des Hinweises auf die Patenterteilung: **10.12.2025**
(21) Anmeldenummer: 20171704.8
(22) Anmeldetag: 04.08.2017
(51) Int. Cl.: G01N 33/08, A01K 45/00, A01K 43/00

(54) **EIERUNTERSUCHUNGSEINRICHTUNG**
ICE EXAMINATION DEVICE
DISPOSITIF D'EXAMEN D' UFS

(30) Priorität: 12.08.2016 DE 102016215127
(43) Veröffentlichungstag der Anmeldung: 09.09.2020
(62) Teilanmeldung aus: 17749176.8
(73) Patentinhaber: HatchTech Group B.V., 6745 XW De Klomp (NL)
(72) Erfinder: Einspanier, Prof. Dr. Almuth, 04103 Leipzig (DE)
(74) Vertreter: Nederlandsch Octrooibureau

(56) Entgegenhaltungen:
- EP-A1- 2 786 656
- EP-A2- 2 505 217
- DE-T2- 69 918 321
- US-A- 1 472 674
- KOENIG R: "ZUR BESCHR[NKUNG DES ANSPRUCHSINHALTS DURCH"BAR"-DERIVATE", MITTEILUNGEN DER DEUTSCHEN PATENTANWAELTE, HEYMANN, KOLN, DE, 1 January 1997 (1997-01-01), pages 62, XP001248632, ISSN: 0026-6884

## Beschreibung

Die vorliegende Erfindung betrifft eine Eieruntersuchungseinrichtung, insbesondere eine Eiergeschlechtsbestimmungseinrichtung. Eine gattungsbildende Eieruntersuchungseinrichtung mit den oberbegrifflichen Merkmalen von Anspruch 1 ist beispielsweise aus der EP 2 786 656 A1 bekannt.

In der EP 2 786 656 A1 beschriebenen Vorrichtung werden mittels Kanülen Flüssigkeitsproben aus einer Anordnung von Eiern entnommen, um anhand von charakteristischen Eigenschaften der jeweiligen Flüssigkeitsprobe die Eier näher charakterisieren zu können.

So wird beispielsweise eine vorherbestimmte Flüssigkeitsmenge an Allantoisflüssigkeit aus den Eiern entnommen, um beispielsweise das Geschlecht der embryonalen Küken zu bestimmen.

Eine Vielzahl von Eiern wird in einer gitterförmigen Anordnung in einer Horde aufgenommen. In der Horde liegen die Eier in deren horizontaler Richtung, d.h. die Rotationsachse des Eis ist senkrecht zur Schwerkraftrichtung ausgerichtet. Da die Eier in der Horde nicht verdrehfest angeordnet sind, werden die Eier in der Vorrichtung der EP 2 786 656 A1 mittels eines Formstempels, der seitlich auf das Ei gedrückt wird, in deren horizontalen Richtung innerhalb der Horde festgelegt und positioniert.

Danach wird die Schale mittels einer dünnen Kanüle punktiert, die an einer Probeentnahmeeinrichtung vorgesehen ist, die von der Seite in das Ei hineinsticht, d.h. senkrecht zur Horizontalrichtung des Eies, um eine Flüssigkeitsmenge zu entnehmen.

Das in diesem Stand der Technik beschriebene Verfahren ist relativ ungenau, weil eine Positionierung der Eier und damit eine Entnahme von Flüssigkeit an einem spezifischen Ort innerhalb des Eis nicht immer gewährleistet ist.

Weiterer relevanter Stand der Technik ist in folgenden Dokumenten offenbart: US 1 472 674 A, EP 2 505 217 A2, EP 2 786 656 A1 und DE 699 18 321 T2.

Ausgehend hiervon schlägt die Erfindung zur Lösung des zuvor genannten Problems eine Eieruntersuchungseinrichtung nach Anspruch 1 vor.

Weitere vorteilhafte Ausgestaltungen sind in den abhängigen Ansprüchen definiert.

Die Zuführeinrichtung ist derart ausgebildet, dass diese die Horde unter einem schrägen Winkel von zwischen 20° und 80° zu einer Ebene senkrecht zur Richtung der Schwerkraft zu der Probeentnahmeeinrichtung zuführt, und optional eine Aushebeeinrichtung vorgesehen ist, mittels welcher das Ei aus der Horde heraushebbar ist und mittels welcher das Ei in eine Probenahmeposition verbringbar ist, in welcher dem Ei mittels der Probeentnahmeeinrichtung die zu entnehmende Flüssigkeitsmenge entnehmbar ist, wobei die Aushebeeinrichtung mittels der Kontrolleinheit steuerbar ist.

Gerade bei der Entnahme von Allantoisflüssigkeit hat sich gezeigt, dass, wenn die Eier nicht senkrecht zur Horizontalen sondern leicht schräg zur Rotationsachse, vorzugsweise schräg von dessen Rückseite angestochen werden, ein Bereich innerhalb des Eies getroffen wird, um in reproduzierbarer Weise vorherbestimmte Flüssigkeitsmengen zu entnehmen kann. Wenn das Ei um den zuvor beschriebenen Winkel in Bezug mit dessen Basis verdreht ist, sammelt sich die Allantoisflüssigkeit in geeignetem Maße an einer wohl definierten Stelle im Ei sammelt, so dass eine gewünschte Menge Allantoisflüssigkeit mittels der Kanüle entnommen werden kann. Besonders vorteilhafte Winkelbereiche sind zwischen 30° und 60°, vorzugsweise 40° bis 50°, insbesondere 45°. Die entsprechenden Winkelbereiche können für sich jeweils obere bzw. untere Grenzen der Eiausrichtung bilden. Zudem hat sich gezeigt, dass unter den zuvor beschriebenen Winkelbereichen eine gute Einstichkraft gewährleistet ist, ohne eine nachhaltige Beschädigung der Schale, welche zu einem Zerbrechen des Eis führt. Da die Eier entweder horizontal oder vertikal in der Horde liegen, insbesondere vertikal, d.h. dass die Längsachse der Eier senkrecht zu der Hordenebene ausgebildet ist, ergibt sich ein entsprechender schräger Winkel, mit welchem die einzelnen Eier von der Aushebeeinheit zu der Probeentnahmeeinrichtung transferiert werden. Von der Aushebeeinheit werden die Eier vorzugsweise senkrecht zu der Horde bzw. der von der Hode gebildeten Fläche, bzw. senkrecht zur schrägen Zuführebene, vorzugsweise durch die Maschen der Horde hindurch herausgebracht und z.B. durch eine entsprechende Einrichtung in dieser Position fixiert . Nachdem ein solches Ei beprobt ist, d.h. eine entsprechende Flüssigkeitsmenge diesem entnommen ist, kann es mit der Aushebeeinrichtung wieder zurück in die Horde verbracht werden und die Horde wird vorzugsweise translatorisch in dem gleichen schrägen Winkel weiter bewegt, um das nächste Ei in der Horde zu beproben. So können im Wesentlichen alle in der schrägstehenden Horde schräg hintereinander angeordneten Eier nacheinander beprobt werden. Das jeweilige Ei wird für die Probeentnahme mittels der Probeentnahmeeinrichtung aus der Horde mittels einer Aushebeeinrichtung herausgehoben und in der Probenahmeposition positioniert. Da die Horden schon schräg zugeführt werden, können die Eier somit während sie einzeln ausgehoben sind, schräg angestochen werden.

Gemäß einer Weiterbildung der Erfindung kann die Aushebeeinrichtung derart konfiguriert sein, dass diese ein mit dieser ausgehobenes Ei in eine vorgegebene Einstechposition rotieren kann. Wenn das Ei mittels der Aushebeeinrichtung aus der Horde gehoben wird kann es vorkommen, dass dieses nicht in einer Position ist, in der idealerweise die Probename stattfindet. Oft kann das Ei durch Rotation, z.B. um dessen Längs- und/oder Querachse rotiert werden um in eine bessere Pobeentnahmeposition gebracht zu werden. Dies kann zum Beispiel durch Rütteln, oder auch durch elektrisch angetriebene Rollen an der Aushebeeinrichtung gewährleistet werden.

Gemäß einer Weiterbildung der Erfindung kann die Aushebeeinrichtung eine mechanische Aushebeeinrichtung sein, oder eine mittels eines Luftstroms betriebene Aushebeeinrichtung, in der das Ei mittels eines Luftstromes ausgehoben wird. Soweit es sich um eine mechanische Aushebeeinrichtung handelt ist eine Ausgestaltung, wie sie weiter unten erläutert wird mit einem Eierstempel und einem Anschlagselement vorteilhaft. Das Ei kann aber auch mittels eines Luftstromes herausgehoben werden, und z.B. gegen ein Anschlagselement gedrückt werden um in die richtige Position gebracht zu werden. In diesem Fall wird ein Schlauch mit einer Öffnung unter dem Ei in der Horde positioniert, und mittels Druckluft das Ei in dem Luftstrom angehoben.

Um die Positionierung und Ausrichtung des zu beprobenden Eies zu ermöglichen ist eine geführte Anhebung des Eies vorteilhaft, diese kann über den Eierstempel, vorzugsweise rotierbar gelagerten Eierstempel geschehen und/oder über einen Luftstrom. Das Ei wird dann von der anderen Seite z.B. mit einem weichen Sauger in Position gehalten. Diese Fixierung kann auch mechanisch und/oder über Luftstrom erfolgen. Zudem kann ventral eine gleitende Führung des Eies in Position erfolgen.

Gemäß einer Weiterbildung der Erfindung kann die Probeentnahmeeinrichtung eine Kanüle aufweisen, welche mit einer Vakuumerzeugungseinrichtung verbunden ist, wobei die aus dem Ei zu entnehmende Flüssigkeitsmenge mittels der Kontrolleinheit über den in der Vakuumerzeugungseinrichtung erzeugten Druck steuerbar ist.Die Kontrolleinheit kann ein Pneumatiksystem steuern, welches zum Beispiel von einem Vakuumschlauch, welcher mit der Kanüle verbunden ist gebildet wird, welcher an einen Vakuumgenerator angeschlossen ist. Über die Steuerung des Druckes kann eine wohl definierte Menge Flüssigkeit aus dem Ei entnommen werden, nachdem die Kanüle schräg in die Eierschale hineingestochen ist. Durch ein solches pneumatisches System und eine Probenahme über eine Vakuumsteuerung können mechanische Elemente, wie sie z.B. bei der Verwendung einfacher Kolbenspritzen als Probeentnahmeeinrichtung vorhanden sind, reduziert werden. Die Probeentnahmeeinrichtung mit der davon ausragenden Kanüle kann zur Probenahme in vertikaler Richtung, d.h. in Schwerkraftrichtung, bewegt werden, wobei die Kanüle bzw. die Spitze der Kanüle die Eierschale durchsticht und die entsprechende vorbestimmte Flüssigkeitsmenge, beispielsweise aus der Allantois, entnimmt. Bevorzugte Kanülenaußendurchmesser sind 0,55 mm; 0,60 mm; 0,65 mm; 0,70 mm; 0,75 mm; 0,80 mm und bevorzugte Kanüleninnendurchmesser sind 0,10 mm; 0,15 mm; 0,20 mm, 0,25 mm, 0,30 mm; 0,35 mm; 0,40 mm; 0,45 mm; 0,50 mm. Die zuvor genannten Werte können jeweils für sich obere bzw. untere Grenzen eines Kanülendickenbereiches bilden.

Gemäß einer Weiterentwicklung der Erfindung kann die Probeentnahmeeinrichtung einen Kanülenrevolver mit mehreren Kanülen aufweisen. Ein Kanülenrevolver kann als drehbare Einrichtung vorgesehen sein, in der, vorzugsweise mittels der Kontrolleinheit, die Kanüle nach jedem Stechen rotiert wird. So kann für jedes Stechen eine neue bzw. andere Kanüle verwendet werden. Hierdurch ist eine schnellere Verfahrensführung gewährleistet. Vorzugsweise kann nämlich, gleichzeitig während die andere Kanüle zum stechen verwendet wird, die Kanüle, mit der zuvor in das Ei gestochen wurde, gereinigt werden. Ein Stechen und Säubern der Nadel in einem Schritt kombinieren kann Zeit bei der Beprobung und Reinigung sparen kann. Der Kanülenrevolver kann mehreren Nadeln insbesondere 6 Nadeln aufweisen.

Gemäß einer Weiterbildung der Erfindung kann die Kanüle zumindest zwei Öffnungen enthalten, mittels welcher die zu entnehmende Flüssigkeitsmenge aus dem Ei entnehmbar ist. Es hat sich gezeigt, dass es manchmal, weil die Position von Membranen innerhalb des Eis auch bei immer gleicher Ausrichtung des Eis nicht dieselbe ist, soweit nur eine einzige Öffnung an der Spitze der Kanüle vorgesehen ist, zu Problemen bei einer Flüssigkeitsentnahme kommen kann, weil eine solche Membran die Öffnung verdeckt. Soweit zumindest zwei Öffnungen, insbesondere genau zwei Öffnungen, vorgesehen sind, ist es unwahrscheinlich, dass alle beiden Öffnungen gleichzeitig verdeckt werden.

Gemäß einer Weiterbildung der Erfindung kann eine überstehende Länge der Kanüle, mit welcher diese vom einen an der Probeentnahmeeinrichtung vorgesehenen Anschlagsfläche, welche bei Probenahme mit dem Ei in Kontakt kommt, abragt, einstellbar sein. Da die in einer Horde angeordneten Eier, z.B. in Abhängigkeit des Legealters der Henne oder der Genetik, unterschiedlich groß sein können, kann beispielsweise in Abhängigkeit der Eiergröße eine unterschiedliche Stichtiefe der Kanüle notwendig sein, um die Flüssigkeitsmenge aus dem Ei zu entnehmen. Die optimale Stichtiefe kann automatisch über bildgebende Verfahren ermittelt werden. Die überstehende Länge der Kanüle wird vorzugsweise, für jedes Ei automatisch angepasst.

Die Probeentnahmeeinrichtung wird, nachdem eine eindeutige Beprobungssituation gefunden wurde, beispielsweise immer solange in vertikaler Richtung auf das Ei gedrückt bis die Kanüle komplett im Ei verschwunden ist. Über eine Regeleinrichtung kann das Eintauchen der Nadel in Flüssigkeit/Allantois bestätigt werden und der definierten Ansaugvorgang gestartet werden.

Es kann auch zum Beispiel detektiert werden sobald das Ei an einer Anschlagsfläche der Probeentnahmeeinrichtung anschlägt. Sobald diese Anschlagfläche von dem Ei berührt wird, was beispielsweise mittels optischer Methoden, wie mittels einer Kamera, oder mittels eines Drucksensors an der Anschlagsfläche gemessen werden kann, instruiert die Kontrolleinheit die Probeentnahmeeinrichtung zum Stoppen deren Bewegung. Die Länge der Kanüle, die aus der Probeentnahmeeinrichtung herausragt bestimmt demnach die Tiefe, in der die Flüssigkeit aus dem Ei entnommen wird. Gemäß einer Weiterbildung der Erfindung kann die übererstehende Länge der Kanüle, mittels der Kontrolleinheit einstellbar sein.. So kannbeispielsweise von der Kontrolleinheit die Kontrolleinheit in Abhängigkeit von Informationen über das jeweilige Ei die Länge der Kanüle einstellen und variieren, und so selektiv für jedes entsprechende Ei in Abhängigkeit dessen Eigenschaften, wie z.B. dessen Dicke, eine Tiefe vorgeben, aus der die Flüssigkeitsmenge entnommen wird.

Alternativ oder zusätzlich, oder auch wenn die überstehende Kanülenlänge nicht veränderbar ist kann die Kontrolleinheit die Probeentnahmeeinrichtung vorbewegen, bis die Kanüle in die Flüssigkeit im Ei (Allantois) eintaucht. Die Kontrolleinheit kann z.B. das Eintauchen der Kanüle in eine Flüssigkeit (Allantois) wahrnehmen und die Vorwärtsbewegung der Probeentnahmeeinheit stoppen, wenn die gewünschte Tiefe erreicht ist. Danach wird z.B. mit dem Saugvorgang begonnen.

Gemäß einer Weiterbildung der Erfindung kann die Probeentnahmeeinrichtung eine Lichtschranke ausweisen, über welche mittels der Kontrolleinheit bestimmbar ist, ob die aus dem Ei entnommene Flüssigkeitsmenge der zu entnehmenden Flüssigkeitsmenge entspricht und nicht durch Luftblasen verändert werden. Die Lichtschranke kann in einer Position vorgesehen sein, in der z.B. der Vakuumschlauch oder eine anderes transparentes Element, durch welches die Flüssigkeit eingesogen wird, durchleuchtet wird. Sobald soviel Flüssigkeit über die Kanüle eingesogen ist, dass die vorherbestimmte Flüssigkeitsmenge erreicht ist, kann durch die Lichtschranke der Meniskus bestimmt werden und somit die Menge an aus dem Ei entnommener Flüssigkeit bestimmt werden. Bevorzugte Entnahmemengen von Flüssigkeit können 1 µl bis 50 µl sein, vorzugsweise 5 bis 30 µl, insbesondere 10 bis 20 µl. Die vorgenannten Werte können für sich jeweils obere und untere Grenzen bilden. Gerade bei der Entnahme solcher kleiner Flüssigkeitsmengen hat sich die Bestimmung der entnommenen Flüssigkeitsmenge mittels Lichtschranken als gute Lösung erwiesen, um eine genaue Entnahmemengenbestimmung durchzuführen und ggf. Luftblasen zu erkennen.

Es können auch mehrere Lichtschranken vorgesehen sein, z.B. Lichtschranken die auf unterschiedlich farbigem Licht basieren. So können z.B. auftretende Probleme bei der Messung der Entnahmemenge minimiert werden, in dem Fall wenn in der entnommenen Flüssigkeit rote Blutkörperchen vorhanden sind.

Gemäß einer Weiterbildung der Erfindung können zumindest zwei Probeentnahmeeinrichtungen vorgesehen sein, welche in einer Probenahmeeinheit zusammengefasst sind, sodass durch jede Probeentnahmeeinrichtung der zumindest zwei gleichzeitig die zu entnehmende Flüssigkeitsprobe jeweils einem Ei der Horde entnehmbar ist, und es können zumindest zwei Aushebeeinrichtungen vorgesehen sein, wobei mittels der jeweiligen Aushebeeinrichtung der zumindest zwei Aushebeeinrichtungen, das der jeweiligen Probenahmeeinheit zugeordnete Ei der Horde aus der Horde aushebbar und in ie Probenahmeposition verbringbar ist. Insbesondere sind mehr als zwei Probeentnahmeeinrichtungen vorgesehen.

So können nach dieser Weiterbildung zumindest zwei Probeentnahmeeinrichtungen vorgesehen sein, denen jeweils eine Aushebeeinrichtung zugeordnet ist, wobei jede Aushebeeinrichtung ein Ei aus der Horde heraushebt und der jeweiligen Probeentnahmeeinrichtung zuführt. So kann für zumindest zwei Eier gleichzeitig eine Probennahme durchgeführt werden. Bei dieser Weiterbildung können auch mehr als zwei Probeentnahmeeinrichtungen und entsprechend mehr als zwei Aushebeeinrichtungen vorgesehen sein, so dass beispielsweise immer drei, vier, fünf oder sogar zehn Eier gleichzeitig beprobt werden können. Die entsprechenden Eier der Horde, die gleichzeitig beprobt werden können, liegen vorzugsweise unmittelbar nebeneinander in einer Reihe der Horde in Querrichtung zu der Schrägstellung der Horde.

So ist es möglich, dass zum Beispiel immer mindestens zwei oder auch mehr Eier nebeneinander mittels einer Vielzahl von entsprechenden Probeentnahmeeinrichtungen beprobt werden können und nachdem die entsprechende Reihe von Eiern beprobt ist, die Horde um eine Einheit verschoben wird, um die in Schrägrichtung darunterliegende Reihe an Eiern zu beproben.

Soweit die Horde nebeneinander (quer zur Schrägstellung) mehr Eier aufweist als Probeentnahmeeinrichtungen vorgesehen sind, kann die Probenahmeeinheit mit den Probeentnahmeeinrichtungen auch in dieser in horizontaler Richtung, also in Richtung der in Reihe nebeneinanderliegenden Eier, mittels der Kontrolleinheit verfahren werden. So können z.B. jeweils zwei bzw. drei bzw. vier bzw. fünf Eier, die in einer Reihe nebeneinander liegen, gleichzeitig beprobt werden. Nach Beproben einer ganzen Eierreihe einer Horde in Horizontalrichtung wird die jeweils nächste Reihe, die in Schrägrichtung darunterliegt, beprobt.

Durch die Ausgestaltung mit zumindest zwei Probeentnahmeeinrichtungen mit einer dieser zugerichteten Aushebeeinrichtung, kann ein schnelles Verfahren bereitgestellt werden, so dass gleichzeitig immer mehrere Eier beprobt werden können.

Gemäß einer Weiterbildung kann die Kontrolleinheit derart ausgebildet sein, dass diese für jede der zumindest zwei Probeentnahmeeinrichtungen die aus dem Ei zu entnehmende Flüssigkeitsmenge individuell in Abhängigkeit von Eierparametern des jeweiligen der Probeentnahmeeinrichtung zugeordneten Eies einstellt.

In Abhängigkeit beispielsweise der Eigröße oder des Bebrütungstags des Eies kann die im Ei enthaltene Menge an Allantoisflüssigkeit abweichen. Somit ist es günstig, in Abhängigkeit von vorher festgestellten Eiparametern die zu entnehmende Flüssigkeitsmenge individuell für jede der zumindest zwei oder mehr Probeeinrichtungen einzustellen. Hierzu kann vorgesehen sein, dass der Druck in den entsprechenden Vakuumschläuchen, die jeder Probeentnahmeeinrichtung zugeordnet sind, individuell eingestellt werden kann, um eine entsprechende Flüssigkeitsmenge zu entnehmen. Auch die Positionierung der vorzugsweise vorgesehenen Lichtschranken zur Bestimmung der entnommenen Flüssigkeitsmenge, kann demnach in Abhängigkeit der zu entnehmenden Flüssigkeitsmenge von der Kontrolleinheit gesteuert werden.

Gemäß einer Weiterbildung der Erfindung kann die Probeentnahmeeinrichtung in Schwerkraftrichtung bewegbar sein. Darüber hinaus kann die Probeentnahmeeinrichtung parallel zu der Fläche innerhalb der die Horde durch die Zuführeinrichtung translatorisch bewegbar ist, und in Richtung quer zur durch die Zuführeinrichtung vorgeschriebene Translationsrichtung der Horde bewegbar sein.

Insbesondere bewegt sich die Probenahmerichtung in Schwerkraftrichtung sowie in Richtung der einzelnen Eier in Reihen nebeneinander quer zur Translationsrichtung der Horde, das heißt parallel zu der Fläche innerhalb der die Horde durch die Zuführeinrichtung translatorisch bewegbar ist, und in Richtung quer zur durch die Zuführeinrichtung vorgeschriebene Translationsrichtung der Horde. So kann z.B. die Kontrolleinheit einen Aktuator ansteuern, der mit der Probeentnahmeeinrichtung zusammenwirkt. Somit ist die Probeentnahmeeinrichtung zumindest in zwei Richtungen innerhalb einer Ebene verschiebbar. Eine solche translatorische Bewegbarkeit kann sowohl für den Fall einer einzelnen Probeentnahmeeinrichtung als auch für den Fall, dass mehrere Probeentnahmeeinrichtungen in einer Probenahmeeinheit zusammengefasst sind, vorgesehen sein. So ist es nämlich möglich, wenn alle Probeentnahmeeinrichtungen in der Probenahmeeinheit zusammengefasst sind, alle Probeentnahmeeinrichtungen in gleichförmiger Weise simultan zu bewegen. Die Bewegungsrichtung in Schwerkraftrichtung ist dazu vorgesehen, die Probeentnahmeeinrichtung auf das jeweilige Ei zuzufahren, um beispielsweise die Kanüle in die Eierschale hineinzudrücken. Die Bewegung in Translationsrichtung ist beispielsweise dafür vorgesehen, Eier, die in Reihen nebeneinander quer zur Zuführungseinrichtung in der Horde liegen, zu beproben. Soweit alle nebeneinander in der Horde liegenden Eier gleichzeitig mit den in einer Probenahmeeinheit zusammengefassten Probeentnahmeeinrichtungen beprobt werden, kann eine solche Bewegbarkeit in Translationsrichtung auch gänzlich weggelassen werden.

Gemäß einer Weiterbildung der Erfindung kann eine Probenaufnahmeeinrichtung vorgesehen sein, welche die von der Probeentnahmeeinrichtung aus dem Ei entnommene aus der Probeentnahmeeinrichtung abgegeben Flüssigkeitsmenge aufnimmt. Eine solche Probeaufnahmeeinrichtung kann jedes Gefäß bzw. auch ein Vlies sein, das der Eieruntersuchungseinrichtung zugeordnet ist, in welches die entnommene Flüssigkeitsmenge aus dem Ei von der Probeentnahmeeinrichtung abgegeben werden kann. Es versteht sich von selbst, dass vorzugsweise jeweils die aus einem Ei entnommene Flüssigkeit in eine entsprechende leere Probeaufnahmeeinrichtung z.B. das leere Gefäß bzw. ein unbenutztes Vlies gegeben wird und demnach so viele Probeaufnahmeeinrichtungen vorgesehen sein können wie Eier in der Horde enthalten sind.

Gemäß einer Weiterbildung der Erfindung können zumindest zwei in einer Einheit zusammengefasste Probenaufnahmeeinrichtungen vorgesehen sein, aus welcher die jeweilige von den zumindest zwei Probeentnahmeeinrichtungen aus dem jeweiligen Ei entnommene Flüssigkeitsmenge abgegeben werden kann. Sobald beispielsweise zwei oder mehr Probeentnahmeeinrichtungen nebeneinander vorgesehen sind, ist es auch vorteilhaft, zwei und bzw. mehr (z.B. eine entsprechende Anzahl) an Probeaufnahmeeinrichtungen nebeneinander anzuordnen, in die jeweils gleichzeitig aus den jeweiligen Probeentnahmeeinrichtungen die Flüssigkeit ausgegeben werden kann. Insbesondere können die Probeaufnahmeeinrichtungen in einer derartigen Anzahl vorgesehen sein, dass sie zumindest der Menge an Eiern in der Horde entsprechen und vorzugsweise können die Probeaufnahmeeinrichtungen auch in derselben Art angeordnet sein wie die Eier in der Horde, z.B. nach Art eines Gitters, angeordnet sind.

Je mehr Probeaufnahmeeinrichtungen als Einheit zusammengefasst sind desto leichter kann diese Einheit in Bezug auf die Probeentnahmeeinrichtung bewegt werden, um die entnommenen Proben darin aufzufangen.

Gemäß einer Weiterbildung können die zumindest zwei in der Einheit zusammengefassten Probenaufnahmeeinrichtungen durch einen Vlies oder eine Titerplatte ausgebildet sein. Das Verfahren zur Bestimmung einer Eigenschaft einer Flüssigkeitsmenge wird insbesondere angewandt, um beispielsweise anhand der Östronsulfatkonzentration der Allantoisflüssigkeit zu bestimmen, inwieweit ein embryonales Gebilde in einem Ei später ein männliches oder ein weibliches Küken wird. Ein solches Verfahren ist ein biochemisches Verfahren, welches mittels einer Doppelantikörpertechnik durchgeführt wird. Auf dem Vlies oder der Titerplatte sind beispielsweise gebundene östronsulfatspezifische Antikörper. Die Reaktion zur Bestimmung der Konzentration an Östronsulfat wird z.B. direkt auf der Probeaufnahmeeinrichtung durchgeführt, d.h. die Konzentration kann indirekt an dem entsprechenden Ort des Vlieses, wo die entnommene Probemenge aufgetragen ist oder auf den entsprechenden Ort der Titerplatte, bzw. sogenannten well-Platte erfolgen. Für weitere Details hierzu sei auf die deutsche Patentanmeldung Nr. 10 2015 226 490.4 verwiesen, deren Offenbarungsgehalt zur Bestimmung der Östronsulfatkonzentration in Allantoisflüssigkeit mittels der Doppelantikörpertechnik zum Offenbarungsgehalt der vorliegenden Anmeldung gemacht wird. Als Probeaufnahmeeinrichtung kann insbesondere eine mittels Direktbeschichtung beschichtete Titerplatte oder ein solcher Vlies verwendet werden. Zur Bestimmung der Konzentration an Östronsulfat sei auf die zuvor genannte Anmeldung verwiesen.

Gemäß einer Weiterbildung der Erfindung kann die Probeentnahmeeinrichtung nach Probeentnahme und Herausziehen der selben aus dem jeweiligen Ei in einer Abgabeposition positionierbar sein, und die Probenaufnahmeeinrichtung, wenn die Probeentnahmeeinrichtung in der Abgabeposition positioniert ist, von einer Probenaufnahmeeinrichtungsruheposition in eine Aufnahmeposition bewegbar sein, in welcher die Probe aus der Probeentnahmeeinrichtung in die Probeaufnahmeeinrichtung abgegeben werden kann.

Der Probenabgabeeinrichtung ist z.B. ein Aktuator zugeordnet, der von der Kontrolleinrichtung derart gesteuert wird, dass nach Probeentnahme mittels der Probeentnahmeeinrichtung und Herausziehen derselben aus dem jeweiligen Ei, wenn die Probeentnahmeeinrichtung in einer Abgabeposition positioniert ist, die Probenaufnahmeeinrichtung von einer Probenaufnahmeeinrichtungsruheposition in eine Aufnahmeposition verfahren wird, in welcher die Probe aus der Probeentnahmeeinrichtung in die Probeaufnahmeeinrichtung abgegeben wird. Somit kann Kontrolleinheit über einen Aktuator die Probeaufnahmeeinrichtung derart zu der Probeentnahmeeinrichtung bringen, dass diese zur Probeabgabe an die Probeaufnahmeeinrichtung mit Ausnahme einer Bewegung in Höhenrichtung, diese nicht translatorisch bewegt werden muss.

Somit ist es möglich, die Probeentnahmeeinrichtung so auszubilden, dass diese beispielsweise nur in zwei Richtungen bewegbar ist, nämlich in senkrechter Richtung und in lateraler Richtung, also in Richtung, in der die Eier nebeneinander in der Horde quer zur schrägen Ausrichtung der Horde angeordnet sind. Trotzdem gibt es auch Varianten in denen es bevorzugt ist, die Probeentnahmeeinrichtung so auszubilden, dass diese aller Richtung en translatorisch bewegbar und/oder auch rotatorisch bewegbar ist. Die Probeaufnahmeeinrichtung wird demnach vorzugsweise immer in eine Position unterhalb der Probeentnahmeeinrichtung bewegt, insbesondere unterhalb des Endes derjenigen Kanüle, aus der die entnommene Flüssigkeitsmenge gegeben werden soll.

Zusätzlich zu der Beweglichkeit in vertikaler Richtung, kann die Probeaufnahmeeinrichtung des Weiteren nicht nur in einer horizontalen Richtung bewegen sondern auch in einer weiteren Horizontalen in der die Eier nebeneinander liegen.

Gemäß einer Weiterbildung der Erfindung kann eine Spüleinrichtung vorgesehen sein, welche nach Probeabgabe in die Probeaufnahmeeinrichtung von einer Spüleinrichtungsruheposition in eine Spülposition bewegbar ist, in welcher die Probeentnahmeeinrichtung mittels eines Druckprofils des in der Vakuumerzeugungseinrichtung erzeugten Druck gespült wird. Nachdem die Flüssigkeitsprobe aus der Probeentnahmeeinrichtung abgegeben ist, wird diese mittels der Spüleinrichtung gespült. Hierzu kann zuerst die Probeaufnahmeeinrichtung wieder in die Probenaufnahmeeinrichtungsruheposition zurückgefahren werden und die Spüleinrichtung von der Spüleinrichtungsruheposition in die Spülposition verfahren werden. Beide Situationen können zeitgleich stattfinden z.B. unter Nutzung des zuvor beschriebenen Kanülenrevolvers.

Die Spüleinrichtung wird demnach vorzugsweise immer in eine Position unterhalb der Probeentnahmeeinrichtung bewegt, insbesondere unterhalb des Endes derjenigen Kanüle, und die Probeentnahmeeinrichtung bzw. die Kanüle wird in die mit Spülflüssigkeit befüllte Spüleinrichtung eingetaucht. Über die Kontrolleinrichtung wird der Druckverlauf in dem Vakuumschlauch derart gesteuert, dass Spüllösung angesogen wird und nachfolgend wieder abgegeben wird. In dieser Spüleinrichtung ist vorzugsweise Alkohol vorgesehen, der das biologische Material aus dem Ei, welches in der Probeentnahmeeinrichtung zurückbleibt, denaturiert. Nachspülen nach dem Spülen mit Alkohol kann z.B. über das pneumatische System geschehen, indem durch die Vakuumschläuche demineralisiertes Wasser gepumpt wird.

Nach Spülen kann auch über das pneumatische System Luft durch die Probeentnahmeeinrichtung geblasen werden, um diese zu trocknen.

Nach Spülen und Trocknen kann die Probeentnahmeeinrichtung wieder verwendet werden, um eine weitere Flüssigkeitsprobe aus dem Ei zu entnehmen.

Insbesondere wird das Verfahren, das in der Eieruntersuchungseinrichtung durchgeführt wird, von der Kontrolleinheit derart gesteuert, dass, soweit lediglich eine Probeentnahmeeinrichtung vorgesehen ist, diese nach Art des Rasterverfahrens aus den einzelnen Eiern eine Flüssigkeitsprobe entnimmt und diese in eine entsprechende Probeaufnahmeeinrichtung abgibt. Nach jeder Probenahme und Abgabe wird ein Spülvorgang durchgeführt. Soweit mehrere Probeentnahmeeinrichtungen nebeneinander vorgesehen sind, kann immer gleichzeitig, z.B. aus zwei, drei, vier, fünf oder zehn Eiern, eine entsprechende Flüssigkeitsprobe entnommen werden. Diese Flüssigkeitsprobe wird dann jeweils in eine entsprechende Anzahl an Probeaufnahmeeinrichtungen abgegeben. Hiernach werden alle Probeentnahmeeinrichtungen gespült. Die Spüleinrichtung kann ein einfaches Flüssigkeitsgefäß sein, in welche alle Probeentnahmeeinrichtungen gleichzeitig eingetaucht werden.

Gemäß einer Weiterbildung der Erfindung kann die Probenaufnahmeeinrichtung und Spüleinrichtung zum Spülen der Probeentnahmeeinrichtung in einer Einheit ausgebildet sind. Wenn die Probenaufnahmeeinrichtung und die Spüleinrichtung als eine Einheit ausgebildet sind, kann diese leicht ausgewechselt werden.

Gemäß einer Weiterbildung der Erfindung kann die Aushebeeinrichtung einen Eierstempel aufweisen, welcher durch eine Masche der Horde hindurch bewegbar ist, um das in dieser Masche gelagerte Ei aus dieser herauszuheben. Zudem kann ein Anschlagselement vorzugsweise mit cranialer Eikopfbegrenzung vorgesehen sein, gegen welches das Ei von dem Eierstempel drückbar ist und welches die Probenahmeposition bestimmt. Der Eierstempel kann ein pufferndes und/oder balancierende Material aufweisen bzw. aus diesem gebildet sein, welches eine Anpassung des Eies in die richtige Richtung ermöglicht. Ein Eierstempel fährt vorzugsweise senkrecht zur Schrägausrichtung der Horde durch eine jeweilige Masche der Horde um das jeweilige Ei aus der Horde heraus zu heben. Während das Ei aus der Horde mittels des Eierstempels herausgedrückt wird, wird dieses angehoben und die Eierbasis an ein Anschlagselement gedrückt, um so das Ei festzusetzen. Das Anschlagselement kann derart ausgeformt sein, dass es eine Rundung aufweist, die entsprechend der Eierbasisrundung entspricht, um das Eier zwischen dem Stempel und dem Anschlagselement zu halten, so dass die Rotationsachse des Eies durch den Eierstempel und das Anschlagselement verläuft. Vorzugsweise steuert die Kontrolleinheit den Eierstempel und das Anschlagselement.

Gemäß einer Weiterbildung der Erfindung kann dem Eierstempel und dem Anschlagselement jeweils zumindest ein Aktuator zugeordnet ist, der über die Kontrolleinheit steuerbar ist. Der jeweilige Aktuator des Eierstempels bzw. des Anschlagelements bewirkt durch Instruktion der Kontrolleinheit eine translatorische Bewegung des Eierstempels und des Anschlagselementes, so dass diese Elemente senkrecht zu der schräg stehenden Horde translatorisch bewegt werden. Hierdurch, ist es auf einfache Weise möglich, das Ei durch die Horde hindurch zu drücken. Dadurch, dass beide Teile, nämlich der Stempel und das Anschlagselement, mit einem Aktuator versehen sind, können diese beiden Elemente derart zusammenwirken, dass sie das Ei zwischen sich greifen und dieses festhalten. So kann das in der Probenahmeposition festgehaltene Ei sicher beprobt werden.

Gemäß einer vorteilhaften Weiterbildung der Erfindung kann eine Positionierbestimmungseinrichtung vorgesehen sein, über welche die Lage des Eies bestimmt wird, wobei die Kontrolleinheit anhand der Daten der Positionierbestimmungseinrichtung eine Positionierung des Eies steuert. Als solche Positionierbestimmungseinrichtung können weiter unten beschriebene Leuchteinheiten, welche vorzugsweise in dem Eierstempel vorgesehen ist, dienen. Als Alternative oder zusätzlich kann auch jedes andere Verfahren oder System, wie beispielsweise ein Kamerasystem oder ein Infrarotsystem vorgesehen sein, um die relative Lage des Eies zu bestimmen und/oder insbesondere die Lage von inneren Abteilungen des Eies, insbesondere der Allantois zu bestimmen.

Anhand der Daten der Positionierbestimmungseinrichtung kann die Kontrolleinrichtung Instruktionen geben, das Ei entsprechend zu positionieren. Die Positionierung findet beispielsweise mittels der Aushebeeinrichtung (mechanisch und/oder per Luftstrom) statt.

Gemäß einer Weiterbildung der Erfindung kann eine Leuchteinheit vorgesehen sein, um das Ei zu durchleuchten um eine Positionsbestimmung des Eis vorzunehmen. Diese Leuchteinheit kann vorzugsweise aus einer oder mehreren LED Lampen gebildet werden. Diese Leuchteinheit durchleuchtet das Ei, was als sogenanntes Schieren bezeichnet wird. So kann die Positionierung des embryonalen Kükens oder auch der Allantois besser bestimmt werden. Es können neben einer einzigen auch mehrere Leuchteinheiten vorgesehen sein.

In Verbindung mit dieser Weiterbildung kann es vorteilhaft sein, dass der Eierstempel mit einem weiteren Rotationselement zusammen arbeitet, der das Ei derart rotiert, dass eine vorgegebene Position des Eies besser erreicht werden kann. Hierbei kann eine Pufferung des Systems erfolgen.

Gemäß einer Weiterbildung der Erfindung kann der Eierstempel mit der Leuchteinheit versehen sein. Vorteilhafterweise kann die Leuchteinheit innerhalb des Eierstempels angeordnet sein.

Gemäß einer Weiterbildung der Erfindung kann des Weiteren eine UV-Lampeneinheit vorgesehen ist, mittels welcher zumindest eines der folgenden Einrichtungen beleuchtet wird um Bakterien und/oder Keime abzutöten: Eier, Probeentnahmeeinrichtung, Spüleinrichtung, Probenaufnahmeeinrichtung, Aushebeeinrichtung. Insbesondere ist es vorteilhaft, die UV-Lampeneinheit als eine Art UV-Röhre oberhalb der schräg stehenden Horde und zwischen der Probeentnahmeeinrichtung und der Probeaufnahmeeinrichtung anzuordnen. Die UV-Röhre ist vorzugsweise quer zur Schrägstellung der Horde angeordnet.

Gemäß einer vorteilhaften Weiterbildung der Erfindung die Kontrolleinheit derart ausgebildet sein, dass eine Beprobung der Eier in der Horde nach Einbringen der Horde mit den Eiern in die Eieruntersuchungseinrichtung automatisiert erfolgt, und nach dem alle Eier der Horde beprobt sind, eine entsprechende Information ausgibt. Das heißt, in der Kontrolleinrichtung ist eine Art Programm niedergelegt, in der ein automatisiertes Verfahren zum Beproben der Eier ausgeführt wird.

Soweit bei der Erfindung auf die Aushebeeinrichtung abgestellt wird, kann diese auch gänzlich weggelassen werden und jede Kombination bzw. Ausgestaltung von Probeentnahmeeinrichtung, Spüleinrichtung, Probenaufnahmeeinrichtung, Horde, Kontrolleinrichtung, Positionsbestimmungseinrichtung, oder auch ein einzelnes dieser Elemente für sich eine Erfindung bilden. Die zuvor genannten Einrichtungen bzw. Elemente können jeweils auch unabhängig von den anderen Bestandteilen der Gesamteinrichtung eine eigene Erfindung bilden.

Weitere vorteilhafte Weiterbildungen der Erfindung ergeben sich aus dem nachfolgend erörterten Ausführungsbeispiel in Verbindung mit der Zeichnung.

In dieser zeigt
- Fig. 1: eine schematische Querschnittsansicht eines Ausführungsbeispiels einer Eieruntersuchungseinrichtung;
- Fig. 2A: eine Aufsicht auf eine Eieruntersuchungseinrichtung, welche ähnlich der aus Fig. 1 ist, wobei die Horde in einer Startposition positioniert ist; und
- Fig. 2B: eine Aufsicht der Eieruntersuchungseinrichtung von Fig. 2A, wobei die Horde nach Beprobung aller Eier in einer Endposition positioniert ist;
- Fig. 3: ein schematisches Schaubild zum Verfahren zur Beprobung eines Eis, wie es in der Eieruntersuchungseinrichtungen aus den Fig. 1 bzw. 2 durchgeführt wird;
- Fig. 4A: einen Ausschnitt aus der Eieruntersuchungseinrichtung aus den Fig. 2, in welcher eine Probenahmeeinheit mit Probeentnahmeeinrichtungen zu sehen ist.
- Fig. 4B und Fig. 4C: eine einzelne Probeentnahmeeinrichtung der Probenahmeeinheit aus Fig. 4;
- Fig. 5A: eine Einheit aus Abgabeeinrichtung und Spüleinrichtung;
- Fig. 5B: die Einheit aus Fig. 5A, wobei eine Schutzabdeckung abgenommen ist;
- Fig. 6: eine Detailansicht des Eierstempels des Ausführungsbeispiels aus Fig. 2; und
- Fig. 7: zeigt ein Beispiel einer Benutzeroberfläche der Software, mittels welchem die Vorrichtung gesteuert wird.

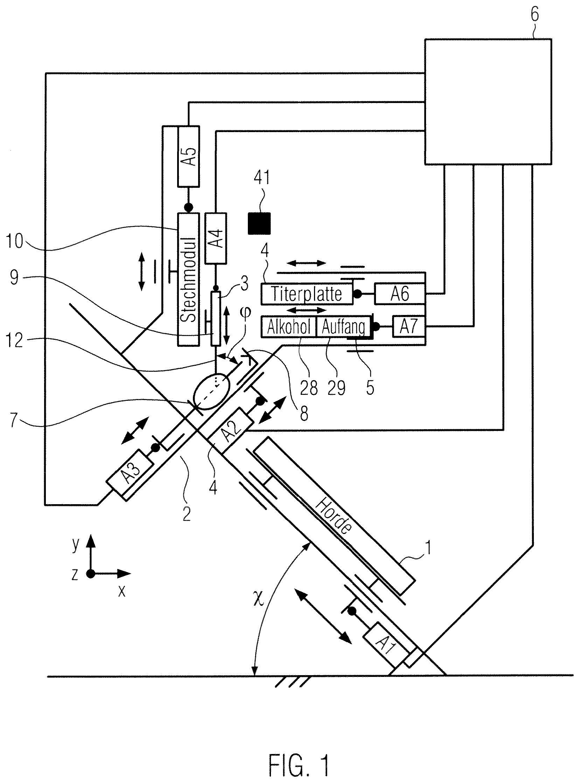

In Fig. 1 ist eine schematische Querschnittsansicht eines Ausführungsbeispiels einer Eieruntersuchungseinrichtung dargestellt.

Die Eieruntersuchungseinrichtung enthält eine Zuführeinrichtung 1, eine Aushebeeinrichtung 2, eine Probeentnahmeeinrichtung 3, eine Probeaufnahmeeinrichtung 4, eine Spüleinrichtung 5 und eine Kontrolleinheit 6. Die Aushebeeinrichtung 2 enthält einen Eierstempel 7 sowie ein Anschlagelement 8. Die Kontrolleinheit 6 steuert, entsprechend Aktuatoren A1, A2, A3, A4, A5, A6, A7, um die zuvor genannten Elemente bzw. Einheiten zu stellen bzw. bewegen.

Alternativ, dazu dass die Aushebeeinrichtung eine mechanische Aushebeeinrichtung mit dem Eierstempel 7 sowie dem Anschlagelement 8 ist kann auch eine mittels eines Luftstroms betriebene Aushebeeinrichtung vorgesehen sein, in der das Ei mittels eines Luftstromes ausgehoben wird. So kann das Ei mittels eines Luftstromes herausgehoben werden, und z.B. gegen ein Anschlagselement gedrückt werden um in die richtige Position gebracht zu werden. In diesem Fall wird ein Schlauch mit einer Öffnung unter dem Ei in der Horde positioniert, und mittels Druckluft das Ei in dem Luftstrom angehoben.

Um die Positionierung und Ausrichtung des zu beprobenden Eies zu ermöglichen eine geführte Anhebung des Eies vorteilhaft, diese kann über den Eierstempel, vorzugsweise rotierbar gelagerten Eierstempel geschehen und/oder über einen Luftstrom. Das Ei wird dann von der anderen Seite z.B. mit einem weichen Sauger in Position gehalten. Diese Fixierung kann auch mechanisch und/oder über Luftstrom erfolgen. Zudem kann ventral eine gleitende Führung des Eies Position erfolgen.

Die Zuführeinrichtung 1 weist einen ersten Aktuator A1 auf, die Aushebeeinrichtung 2 aus dem Eierstempel 7 und dem Anschlagelement 8 einen zweiten Aktuator A2, der das Anschlagselement 8 stellt und einen dritten Aktuator A3, der den Eierstempel 7 stellt. Über einen in Fig. 1 dargestellten vierten Aktuator A4 wird die in dieser Figur dargestellte Spritze 9 aufgezogen. Eine Grundplatte 10 mit der daran befestigten Spritze 9 bildet eine Probenahmeeinheit, wobei die Spritze 9 die Probeentnahmeeinrichtung bildet. Die Probenahmeeinheit als Ganzes, wird über einen fünften Aktuator A5 gestellt. Der Probeaufnahmeeinheit 4 ist ein sechster Aktuator A6 zugeordnet sowie der Spüleinrichtung 5 ein siebter Aktuator zugeordnet.

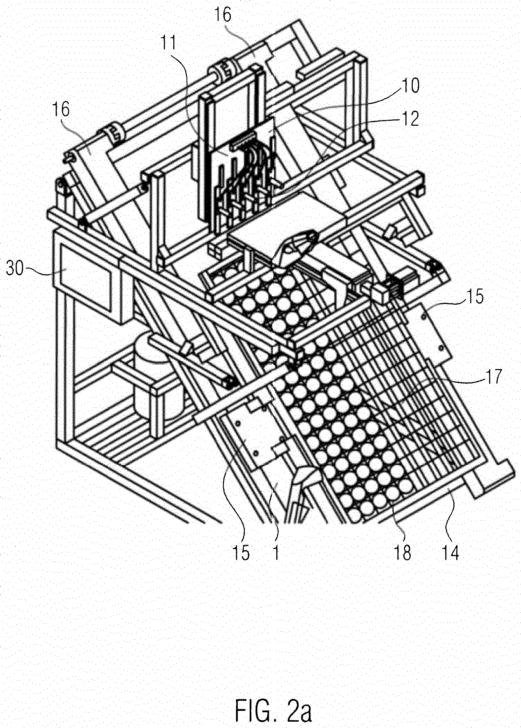

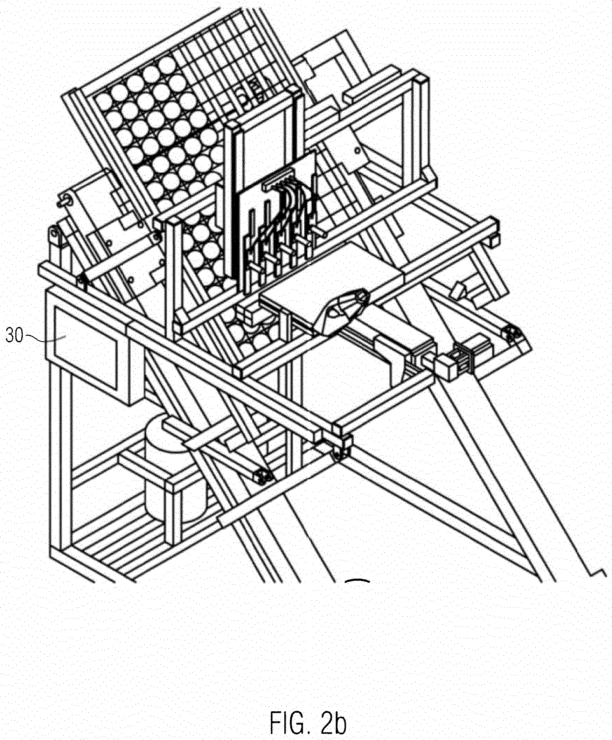

In Fig. 2A eine Aufsicht auf die in Fig. 1 gezeigte Eieruntersuchungseinrichtung, wobei eine Horde 13 in einer Startposition positioniert gezeigt ist. Ein Unterschied der Eieruntersuchungseinrichtung aus Figur 2 zum ersten Ausführungsbeispiel aus Figur 1 ist, dass an der Probenahmeeinheit keine simple Spritze als Probeentnahmeeinrichtung 9 vorgesehen ist, sondern die Probenahme über an Vakuumschläuche 11 angeschlossene Kanülen 12 stattfindet, wobei jede Kanüle mit daran angeschlossenem Vakuumschlauch 11 eine Probenahmeeinheit 9 bildet, und demnach fünf Probenahmeeinheiten in der Probenahmeeinheit vorgesehen sind. Es können zwischen einer und einer Vielzahl von Probenahmeeinheiten jede beliebige Anzahl, insbesondere 5, 10, 15, Probenahmeeinheiten 9 zu einer Einheit zusammengefasst sein.

In Fig. 2A ist eine Startposition dargestellt, nachdem die Horde 13 wie vorliegend, zum Beispiel zur Hälfte mit Eiern 19 gefüllt ist, in die Zuführeinrichtung 1 eingesetzt wurde. Die Zuführeinrichtung 1 ist im vorliegenden Fall als eine Art Rahmen 14 ausgebildet, in welchem die Horde 13 derart eingesetzt werden kann, dass mit deren Seitenbegrenzungswänden im Wesentlichen bündig in dem Rahmen 14 anliegen.

Dieser Rahmen weist an zwei in Querrichtung der Vorrichtung gegenüberliegenden Seiten einen Schlitten 15 auf, der auf entsprechenden Schienen 16 verschiebbar gelagert ist und so mittels des in Fig. 2A nicht gezeigten ersten Aktuators A1 über die Kontrolleinheit 6 von der in Fig. 2A gezeigten Startposition in eine in Fig. 2B gezeigte Endposition verschoben werden kann.

Um nachfolgend die einzelnen Richtungen leichter zu identifizieren, soll die Schwerkraftrichtung, d.h. die Richtung in Fig. 1, 2A und 2B von oben nach unten als y-Achse, die Achse senkrecht zur dieser Vertikalen, d.h. zur y-Achse und quer zur Horde 13 als z-Achse sowie die Achse, in der sich die Schrägstellung der Horde 13 erstreckt, als x-Achse bezeichnet werden. Ein entsprechendes Koordinatensystem ist in Fig. 2A dargestellt. Ein entsprechendes Koordinatensystem ist ebenfalls in Fig. 1 dargestellt. Der erste Aktuator A1 gewährleistet eine translatorische Bewegung der Zuführeinrichtung 1 in der y-x-Ebene. Der zweite Aktuator A2, und der dritte Aktuator A3, gewährleistet eine translatorische Bewegung der einzelnen Elemente der Aushebeeinrichtung innerhalb der y-x- Ebene und senkrecht zu der Bewegung des ersten Aktuators A1. Der vierte bzw. Fünfte Aktuator A4, A5 gewährleistet eine translatorische Bewegung der entsprechenden Elemente (Probeentnahmeeinrichtung bzw. Probeentnahmeeinheit) in y- Richtung. Der siebte bzw. achte Aktuator A7, A8 gewährleistet eine translatorische Bewegung der entsprechenden Elemente in x-Richtung.

Die Horde 13 ist als eine Art Gitter 17 ausgebildet mit gleich großen Maschen, wobei in jeder Masche jeweils ein einziges Ei 18 gehalten werden kann. Da die Maschen lediglich von streifenartigen hochkant stehenden Wänden begrenzt werden, liegt jedes Ei 18 mit seiner Spitze nach unten in der jeweiligen Masche. Das heißt, in Fig. 2A ist eine Basisseite der Eier 18 dargestellt. Die Eier 18 liegen in einer Matrix, wobei im vorliegenden Beispiel jeweils fünf Eier 18 in Querrichtung (z-Achse) nebeneinander und 17 Eier in Längsrichtung, bzw. Schrägrichtung (x-Achse) hintereinander liegen. Im Folgenden sei als nebeneinander bei den Eiern immer die Richtung der z-Achse und hintereinander immer die Translationsrichtung (x-Achse) der Zuführeinrichtung 1 bezeichnet.

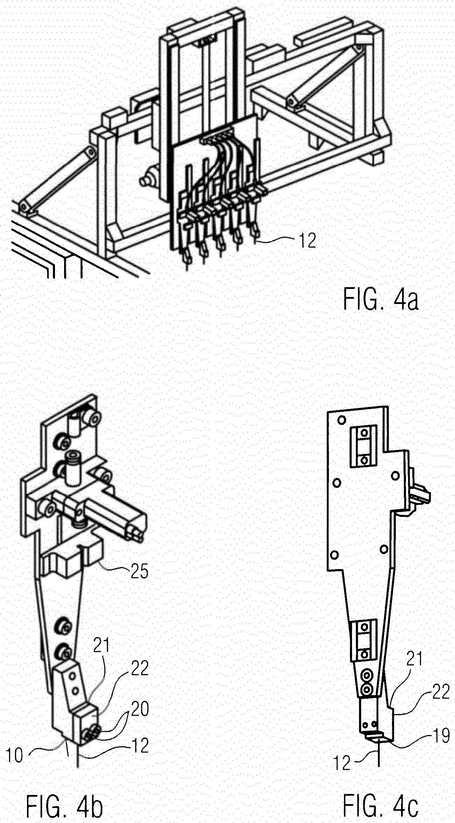

Im vorliegenden Ausführungsbeispiel sind an der Grundplatte 10 fünf Probeentnahmeeinrichtungen 3 vorgesehen wodurch die Probenahmeeinheit gebildet wird. Jede der Probeentnahmeeinrichtungen 3 ist modular aufgebaut und nachfolgend in Bezug auf die Fig. 4A - 4C näher beschrieben. Wie in Fig. 4B und C dargestellt, ist jede Probeentnahmeeinrichtung 3 als Modul ausgebildet, welches beispielsweise über Schraubverbindungen an der Grundplatte 10 befestigt werden kann. An einer im an der Grundplatte 10 montierten Zustand zum Ei hingerichteten Seite des Moduls (im Folgenden Unterseite des Moduls) ist eine Anschlagsfläche 19 vorgesehen, aus welcher die Kanüle 12 herausragt. Die Kanüle 12 ist mittels einer Art Klemmverbindung zwischen den Plattenelementen 21, 22 des Moduls gehalten, indem das Plattenelement 22 mittels den in Fig. 4B dargestellten zwei Befestigungselementen 20, welches im vorliegenden Fall Imbusschrauben sind, zusammengepresst wird. Wie in Fig. 4A dargestellt, ist rückseitig an jeder Kanüle 12, die vorzugsweise eine dünne Metallkanüle mit einer scharfen Spitze ist, jeweils ein Vakuumschlauch 11 angeschlossen. Die Vakuumschläuche 11 sind an ein in den Figuren nicht näher dargestelltes pneumatisches System angeschlossen. Mit dem im pneumatischen System erzeugten Unterdruck kann nach Einstechen eines jeweiligen Eies mit der Kanüle 12 eine vorherbestimmte Flüssigkeitsmenge dem Ei 18 entnommen werden.

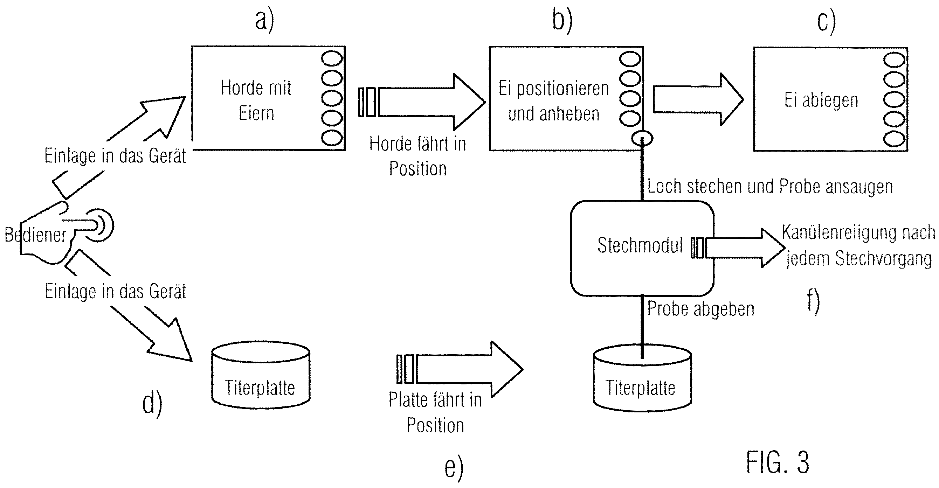

Das Verfahren zur Probenahme wird nachfolgend in Verbindung mit Fig. 3 näher beschrieben.

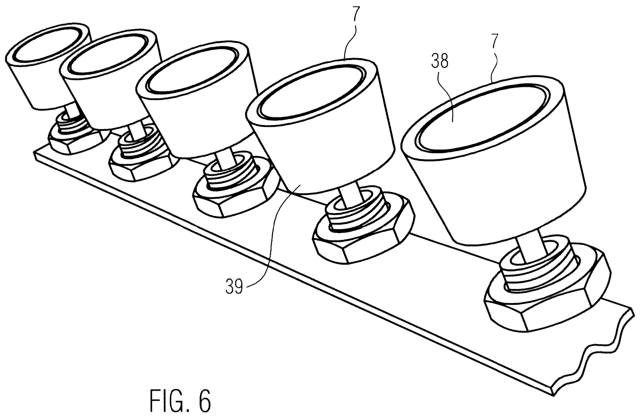

Nachdem von einer Bedienperson, die mit den Eiern 18 bestückte Horde 13 in die Zuführeinrichtung 1 eingelegt ist (Schritt a) in Fig. 3), wird das Ei 18 positioniert. Hierfür wird über die Kontrolleinheit 6 die Zuführeinrichtung 1 derart bewegt, dass die in Translationsrichtung der Horde (x-Achse) gesehen erste Eierreihe in auf Höhe der Probeentnahmeeinrichtung 3 (vergleiche Fig. 2A) zum Stehen kommt (Schritt b) in Fig. 3). So sind in dieser Position im vorliegenden Beispiel fünf Eier unter den jeweiligen 5 Probeentnahmeeinrichtungen 3 der Probenahmeeinheit angeordnet. Mit den in Fig. 6 gezeigten Eierstempeln 7, werden die fünf Eier gleichzeitig im Wesentlichen senkrecht zu der Horde 13, das heißt senkrecht zu der durch den Rahmen 14 der Hode 13 gebildeten Fläche, bzw. senkrecht zur schrägen Zuführebene, durch die Maschen der Horde 13 hindurch herausgedrückt, um so aus der Horde 13 ausgehoben zu werden und gegen das in Fig. 1 schematisch dargestellte Anschlagselement 8 gedrückt.

So werden die Eier mit deren dickeren Rückseite in einem schrägen Winkel zwischen 20 und 80° ausgerichtet aus der Horde gehoben. Der Winkel φ (vergleiche Fig. 1) zwischen der Rotationsachse (Längsrichtung) des Eies 18 und der Vertikalrichtung (y-Achse), d.h. der Richtung, in der die Kanüle 12 in das Ei 18 gestochen wird (im vorliegenden Fall die Richtung in der die Probeentnahmeeinheit in deren vertikalen Richtung bewegbar ist), entspricht aufgrund der Trigonometrie dem Winkel χ, unter dem die Zuführeinrichtung 1 die Horde 13 schräg zuführt (vergleiche Fig. 1). Der Winkel φ zwischen der Rotationsachse (Längsrichtung) des Eies 18 und der Kanüle 12 entspricht dem Winkel χ zwischen der x-Achse (im vorliegenden Fall die Bodenfläche) und der Translationsrichtung der Zuführeinrichtung 1, da ihre Schenkel paarweise aufeinander senkrecht stehen. Die Ausheberichtung der Eier ist senkrecht zur Hordenebene, welche der Translationsebene entspricht.

Nach Ausheben des Eies 18 wird dies nun zwischen dem Eierstempel 7 und dem Anschlagelement 8 festgehalten.

Danach wird im vorliegenden Beispiel die komplette Grundplatte 10 mit den sich daran befestigten Probeentnahmeeinrichtungen 3, das heißt die Probenahmeeinheit, auf der y-Achse heruntergefahren, damit die fünf Kanülen 12 gleichzeitig in die fünf nebeneinanderliegenden aufgehobenen Eier 18 stechen.

Sobald die Anschlagsfläche 19 der jeweiligen Probenahmeeinheit 3 an der Schale des jeweiligen Eis 18 anschlägt, wird dies von der Kontrolleinheit 6 detektiert und die Probenahme wird gestartet.

Hierzu wird, nachdem das Loch in dem Ei 18 durch Einstechen der jeweiligen Kanüle 12 erzeugt wurde, ein Vakuum in den Vakuumschläuchen 11 erzeugt, sodass eine entsprechende Flüssigkeitsmenge aus dem Ei gesogen wird.

Die voreingestellte Sollmenge der dem Ei entnommenen Flüssigkeit wird über eine schematisch in Fig. 4B dargestellte Lichtschranke 25 detektiert. Das heißt, sobald der Flüssigkeitsmeniskus in dem Vakuumschlauch 11 die Lichtschranke 25 erreicht, wird der Druck konstant gehalten.

Hiernach wird die Probenahmeeinheit wieder zurückgezogen (in vertikaler Richtung; y-Achse) und die Kanülen 12 aus den Eiern herausgezogen. Die Spitzen der Kanülen 12 sollen hierbei vorzugsweise in einer derartigen Höhe zum Halten kommen, dass die Probeaufnahmeeinrichtung 4 bzw. die Spüleinrichtung 5 mittels des in Fig. 1 dargestellten sechsten bzw. siebten Aktuators A6, A7, wie die Pfeile in Fig. 1 vorgeben, aus der mit Bezugszeichen 26 versehenen Probeabgabe und Reinigungsgruppe translatorisch unter die Kanülenspitze bewegt werden kann.

Im vorliegenden Beispiel handelt es sich um eine sogenannte Titerplatte, bei der einzelne Probeaufnahmeeinrichtungen 4, d.h. beispielsweise Kavitäten, nebeneinander und hintereinander angeordnet sind, dass sich eine Matrix aus Probeaufnahmeeinrichtungen 4 ergibt.

Mittels Steuerung durch die Kontrolleinheit 6 wird, nach translatorischer Bewegung der Titerplatte (in x-Achse) unter die Kanülenenden (Schritt e) in Fig. 3)., nun entsprechender Druck auf die Vakuumschläuche 11 gegeben, so dass die aus den Eiern 18 entnommene Flüssigkeitsmenge aus den Probenahmeeinheiteneinrichtungen 3 in die entsprechende darunter vorgesehene Probeaufnahmeeinrichtung 27 abgegeben wird. Dies geschieht im Wesentlichen gleichzeitig, so dass, nachdem im vorliegenden Beispiel fünf Flüssigkeitsmengen aus fünf Eiern 18 gleichzeitig entnommen wurden, auch wieder von fünf Flüssigkeitsproben, eine jeweils in die entsprechende Probeaufnahmeeinrichtung abgegeben werden. Die entsprechende Titerplatte (Schritt d) in Fig. 3) wird zuvor von der Bedienperson, ebenso wie die Horde mit den Eiern in die Eieruntersuchungseinrichtung eingelegt.

In jeder der Probenahmeaufnahmeeinrichtungen 3 wird dann eine Analyse durchgeführt und im vorliegenden Fall wird zum Beispiel die Konzentration an Östronsulfatkonzentration in der aus den Eiern 18 entnommenen Allantoisflüssigkeit bestimmt, um daraus auf das Geschlecht der embryonalen Küken schließen zu können. Ein solches Verfahren zur biologischen Bestimmung der Östronsulfatkonzentration zum Identifizieren von weiblichen und männlichen Hühnerembryonen ist in der deutschen Patentanmeldung Anmeldenummer 10 2015 226 490.4 beschrieben, deren Offenbarungsgehalt in dieser Hinsicht durch diesen Verweis einbezogen wird. Anhand einer kolorimetrischen Messung kann indirekt die Konzentration des Östronsulfats bestimmt werden.

Nachdem die einzelnen entnommenen Flüssigkeitsmengen aus den Eiern in die Probeaufnahmeeinrichtungen 4 abgegeben sind, werden die fünf von der Aushebeeinrichtung gehaltenen Eier 18 wieder zurück in die Position der Horde gelegt (Schritt c) in Fig. 3). Dies kann alternativ auch schon unmittelbar nach Flüssigkeitsentnahme und vor Probenabgabe geschehen.

Die Titerplatte, d.h. die Matrix von Probeaufnahmeeinrichtungen 4 wird wieder zurück in deren in Fig. 2B bzw. 1 gezeigte Grundposition in der Probeabgabe und Reinigungsbaugruppe bewegt.

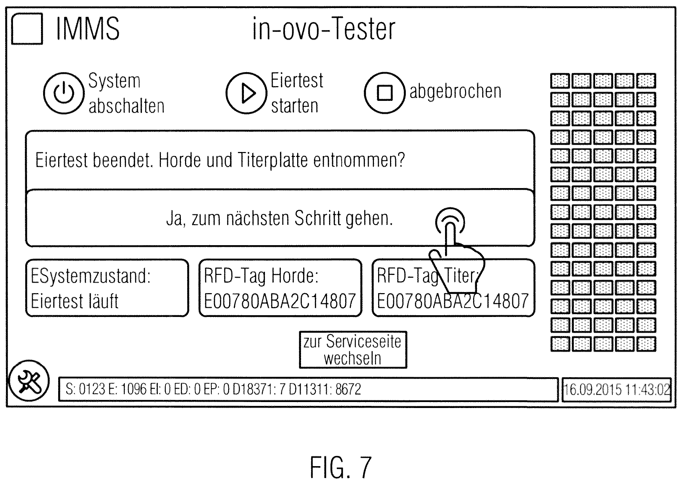

Zur Reinigung der Kanülen 12 wird nunmehr die Spüleinrichtung 5, die im vorliegenden Beispiel aus einer Wanne 28, die mit Alkohol gefüllt ist, und einem Auffanggefäß 29 besteht, mittels des siebten Aktuators A7 (vgl. Fig. 1), wie in Fig. 7 gezeigt von der Grundposition innerhalb der Probeabgabe und Reinigungsbaugruppe in eine in der Figur nicht dargestellte Spülposition verbracht, in welche die Spüleinrichtung 7 im Wesentlichen unterhalb der Kanülen 12, d.h. unterhalb der jeweiligen Probeentnahmeeinrichtung 3 zum stehen kommt. Die Reinigung der Kanülen kann nach jedem Stechvorgang stattfinden (Schritt f) in Fig. 3).

In einem ersten Schritt wird die Probenahmeeinheit in vertikaler Richtung nach unten, d.h. auf der y-Achse verfahren, um die Kanülen 12 mit deren Spitzen in die Wanne 28 mit Alkohol zu tauchen.

Über die Kontrolleinheit 6 wird nun ein wenig Alkohol aus der Wanne 28 in die Kanüle gesogen, um die biologischen Rückstände zu denaturieren. Hiernach wird die Probenahmeeinheit über den fünften Aktuator A5 in vertikaler Richtung (entlang der y-Achse) nach oben zurückgezogen und das Auffanggefäß 29 wird von der Spülposition in eine Spülflüssigkeitsabgabeposition gefahren, so dass das Auffanggefäß 29 unter der Probeentnahmeeinrichtung 3 angeordnet ist. Hiernach wird über die Vakuumschläuche demineralisiertes Wasser, welches in einem in den Figuren nicht dargestellten Behälter der Vorrichtung gelagert ist in die Kanülen 12 eingeführt und die Kanülen 12 werden mit diesem Wasser nachgespült, welches im Auffanggefäß 29 aufgefangen wird.

Nach dem Spülen findet vorzugsweise eine Trocknung der Kanülen und/oder der Vakuumschläuche statt. Dies kann beispielsweise durch separate Luftdüsen geschehen, die in der Untersuchungseinrichtung vorgesehen sind, und die Kanülen 12 von außen beblasen. Im vorliegenden Ausführungsbeispiel wird mittels der Pneumatik Luft durch die Vakuumschläuche 11 und damit die Kanülen 12 beblasen.

Nachdem die Probeentnahmeeinrichtungen gereinigt sind, wird die Spüleinrichtung 5 wieder in die Grundposition, wie sie in Fig. 1 dargestellt ist, zurückgezogen.

Nachfolgend wird über die Kontrolleinheit 6, die Zuführeinrichtung 1 derart gestellt, dass die Horde 13 um eine Reihe nach in Translationsrichtung nach vorne schräg verschoben wird, um somit die nächste Reihe von vorliegend fünf Eiern 18 gleichzeitig zu beproben.

Dieses vorgeschriebene Verfahren wird so oft wiederholt, bis in Längsrichtung alle hintereinander liegenden Eier der Horde beprobt sind.

Die Position in die Matrix an Probeaufnahmeeinrichtungen 4 in der die entnommene Flüssigkeitsmenge auf die Titerplatte gegebenen wird entspricht der Position der Eier in den Horden. So kann jedem Ei in der Hode eine einzige Probenaufnahmeeinrichtung eindeutig zugeordnet werden.

Die gesamte Eieruntersuchungseinrichtung kann über einen berührungsempfindlichen Computerbildschirm, der mit der Kontrolleinheit 6 verbunden ist bedient werden. Der Computerbildschirm ist in den Fig. 2A und B mit Bezugszeichen 30 versehen.

Ein Beispiel einer Benutzeroberfläche der Software, mittels welchem die Vorrichtung gesteuert wird, welche auf dem Computerbildschirm 30 angezeigt wird, ist in Fig. 7 dargestellt. In Fig. 7 ist ein Zustand der Benutzeroberfläche dargestellt, in der das Beprobungsverfahren einmal zu Ende durchgeführt wurde. Deshalb sind in der auf der rechten Seite dargestellten schematischen Anordnung der Eier in den Horden die Quadrate ausgefüllt gezeichnet.

Soweit ein jeweiliges Ei noch nicht beprobt ist, werden diese Quadrate nicht ausgefüllt sondern lediglich durch eine umlaufende Linie gezeichnet.

Zudem ist in Fig. 7 zu sehen, dass jede Horde und die entsprechende Titerplatte, in welche die Proben abgegeben werden, eindeutig einander zuordenbar ist. Im vorliegenden Beispiel ist dies über einen RFID Tag gewährleistet, der jeweils an einer Hode und an der Titerplatte vorgesehen ist. Einer im RFID Tag hinterlegten Hordennummer kann entsprechend eine Titerplattennummer zugeordnet werden.

Sobald alle Eier der Horde wie gewünscht beprobt sind, wird die Charakterisierung üblicherweise nicht innerhalb der erfindungsgemäßen Vorrichtung durchgeführt sondern in einer separaten Vorrichtung. Das heißt die mit Proben gefüllten Titerplatten werden aus der Vorrichtung entnommen und zur Bestimmung der Östronsulfatkonzentration einer entsprechenden Vorrichtung zugeführt. Dadurch, dass die Anordnung der Eier in Horden bekannt ist und die Position, in welcher Probeaufnahmeeinrichtung die entsprechende Flüssigkeit von einem jeweiligen Ei der Horde abgegeben ist, kann auch später noch bestimmt werden, welches Ei in der Horde welchem Ergebnis auf der Titerplatte entspricht.

Da sich in Abhängigkeit des Legehennenalters, auch die Eiergröße variieren kann, ist es oft schwierig alle Eier mit derselben Kanülenlänge (Überstand der Kanüle von der Anschlagsfläche 19) zu beproben. Denn die überstehende Kanülenlänge bestimmt im die Tiefe, aus welcher die entsprechende Flüssigkeitsmenge aus dem Ei 18 entnommen wird. Um beispielsweise Allantoisflüssigkeit zu entnehmen, muss immer eine vorherbestimmte Tiefe unter einem zuvor beschriebenen Winkel erreicht werden.

Die überstehende Länge der Kanüle, kann mittels der Kontrolleinheit einstellbar sein. So kann beispielsweise von der Kontrolleinheit die Kontrolleinheit in Abhängigkeit von Informationen über das jeweilige Ei die Länge der Kanüle einstellen und variieren, und so selektiv für jedes entsprechende Ei in Abhängigkeit dessen Eigenschaften, wie z.B. dessen Dicke, eine Tiefe vorgeben, aus der die Flüssigkeitsmenge entnommen wird.

Alternativ oder zusätzlich, oder auch wenn die überstehende Kanülenlänge nicht veränderbar ist kann die Kontrolleinheit die Probeentnahmeeinrichtung vorbewegen, bis die Kanüle in die Flüssigkeit im Ei (Allantois) eintaucht. Die Kontrolleinheit kann z.B. das Eintauchen der Kanüle in eine Flüssigkeit (Allantois) wahrnehmen und die Vorwärtsbewegung der Probeentnahmeeinheit stoppen, wenn die gewünschte Tiefe erreicht ist. Danach wird z.B. mit dem Saugvorgang begonnen.

Die Längeneinstellung der Kanülen kann auch unabhängig von der Eieruntersuchungseinrichtung für sich eine Erfindung bilden.

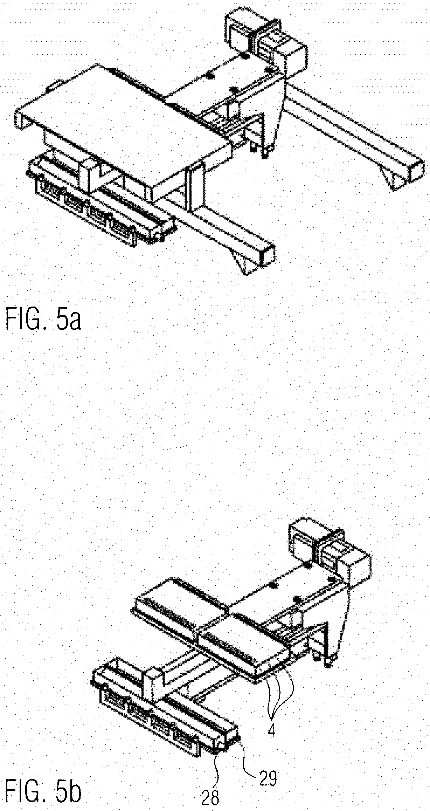

In Fig. 5A und 5B ist eine Detailansicht der Probenabgabe und Reinigungsbaugruppe 26 zu sehen. Dieses weist eine Abdeckung 35 zum Schutz vor Umgebungseinflüssen wie Staub, eine erste Abstellfläche 36 auf, auf welcher die Spüleinrichtung 5 aufliegt, und eine zweite Abstellfläche 37 auf welcher die in der Titerplatte zusammengefassten Probeaufnahmeeinrichtungen 4 abgestellt sind. Wie in Fig. 5B dargestellt, sind vorliegend zwei Titerplatten vorgesehen. Anstelle der Titerplatte kann auch ein Vlies vorgesehen sein, in der die entnommene Probe an verschiedenen Stellen abgegeben wird.

Jede der beiden Abstellflächen sind jeweils für sich an einem translatorisch bewegbaren Arm befestigt. Die beiden Arme können sich über den siebten Aktuator A7, die Spüleinrichtung 5 bzw. die Titerplatte/Vlies unabhängig voneinander von der Grundposition in die entsprechende Abgabeposition für die Probeaufnahmeeinheit bzw. für Spül- oder Ausblasposition für die Spüleinheit bewegen.

Die Reinigungsbaugruppe 26 kann auch unabhängig von der Eieruntersuchungseinrichtung für sich eine Erfindung bilden.

Wie in Fig. 6 dargestellt, sind die Eierstempel 24 als leicht konische Kunststoffhülsen 39 ausgebildet, die in deren Mitte ein Element 38 aus regulierendem und ausgleichendem Material, z.B. kompressiblem Material aufweisen. Die Eierstempel 24 werden über die unterseitig an diesen angebrachten Kolben von dem in Fig. 1 dargestellten dritten Aktuator A3 translatorisch bewegt. Hierbei wird die Spitze der Eier mit dem Element 38 aus regulierendem und ausgleichendem Material in Kontakt gebracht und die Eier werden von den konischen Kunststoffhülsen 39, welche als Eikopf umfassenden Ansauger ausgebildet sind an deren Umfangsrichtung unterstützend gehalten.

Um eine Positionierung der Eier besser bestimmen zu können, kann anstelle des Elements 38 aus regulierendem und ausgleichendem Material auch Leuchtelemente, beispielsweise eine LED Einheit eingebracht sein, mittels derer ein sogenanntes Schieren durchgeführt wird. Mittels dieses Durchleuchtens der Eier können Positionen von verschiedenen Gruppierungen innerhalb des Eis besser bestimmt werden. So kann beispielsweise eine Nachjustierung des Eis stattfinden, um beim Einstechen die Allantois besser zu treffenDes Weiteren ist es denkbar, dass anstelle der mechanisch mittels der Befestigungselemente höhenverstellbaren Kanülen 12 an den Probenahmeeinheiten 3 jeweils weitere Aktuatoren vorgesehen sind, die ein automatisches Verstellen der Kanülenlänge über die Kontrolleinheit 6 ermöglichen.

Es ist vorteilhaft, beispielsweise die Größe und Form jedes Eies 18 mittels einer Oberservationseinrichtung, wie beispielsweise einer Kamera, zu bestimmen, um so mittels Bildverarbeitungsmethoden Informationen über die Eigenschaften bzw. Position jedes Eies zu gewinnen. Diese Informationen können an die Kontrolleinrichtung 6 weitergegeben werden, die dann entsprechend über die Aktuatoren der Probeentnahmeeinrichtungen für die jeweilige Kanüle 12 der jeweiligen Probenahmeeinheit 3 die Länge einstellt. Eine Verbindung mit Kamera und Lichtquelle zur Ausrichtung des Eies ist denkbar.

Im vorliegenden Ausführungsbeispiel werden bei fünf Eiern gleichzeitig Proben entnommen. Die Probeentnahme ist nicht auf fünf Eier beschränkt. Für die vorliegende Erfindung reicht eine einzige Probeentnahmeeinheit aus. Vorzugsweise können jedoch zumindest zwei insbesondere bis zu zehn Eier gleichzeitig beprobt werden.

Auch wenn bei dem zweiten Ausführungsbeispiel eine Vakuumsteuerung der Probenahme durchgeführt wird, kann alternativ, wie im ersten Ausführungsbeispiel auch eine einfache Spritze vorgesehen sein, die über einen mechanischen Kolben betätigt werden kann.

Bei der vorliegenden Ausgestaltung der Eieruntersuchungseinrichtung ist es besonders effektiv möglich, die Eier in einer automatisierten Weise unter einem schrägen Winkel einzustechen, wobei vorzugsweise die Probeentnahmeeinrichtung, d.h. die daran angeordnete Kanüle, in senkrechter Richtung, d.h. in Schwerkraftrichtung eingestochen wird, wobei jedes Ei für sich leicht schräg mit einem Winkel von 20° bis 80° ausgerichtet wird.

Hierzu werden zum einen die in den Horden gelagerten Eier mitsamt der Horde über die Zuführeinrichtung schräg zur Schwerkraftrichtung zugeführt und dann aus der jeweiligen Masche der Horde mittels der Aushebeeinrichtung senkrecht zu dieser schrägen Fläche ausgehoben.

Die Eier, werden in der Aushebeeinrichtung, vorliegend mittels Kunststoffhülsen 39, welche als Eikopf umfassenden Ansauger gebildet sind, und dem Anschlagselement einzeln seitlich, vorne und hinten und unabhängig von der Horde festgehalten, um ein selektives genaues Stechen unter einem vordefinierten Winkel zu gewährleisten und Eier zu beproben..

Dadurch, dass die Kanüle 12 in vertikaler Richtung (in Schwerkraftrichtung) eingestochen wird, entspricht der Winkel, mit dem die Zuführeinrichtung 1 die Eier zuführt, im Wesentlichen der Schrägstellung der Eier in Bezug auf die Kanüle 12.

Besonders vorteilhafte Winkelbereiche sind zwischen 30° und 60°, vorzugsweise zwischen 40° und 50°, insbesondere 45°. Insbesondere ist es vorteilhaft, den Winkel so festzulegen, dass dieser der Winkel zwischen der Kanüle und der Rotationsachse des Eies von der Eibasis aus gesehen und nicht der Spitze aus gesehen ist.

Wenn das Ei um genau den zuvor beschriebenen Winkel in Bezug mit dessen Basis verdreht ist, hat sich gezeigt, dass sich die Allantoisflüssigkeit in geeignetem Maße an einer wohl definierten Stelle im Ei sammelt, so dass wohldefinierte Menge Allantoisflüssigkeit mittels der Kanüle entnommen werden kann.

Um dieses Verfahren noch reproduzierbarer zu machen, ist es vorteilhaft, dass die verwendete Spitze der Kanüle zumindest zwei Öffnungen hat. Denn es hat sich herausgestellt, dass, soweit nur eine Öffnung verwendet wird, nicht unter allen Bedingungen eine ideale Probeentnahme erreicht wird. Auch mehr als zwei Öffnungen in der Kanüle sind möglich. Unter manchen Umständen kann auch eine einzige Öffnung in der Kanüle vorteilhaft sein.

Vorteilhafterweise kann bei der Untersuchungseinrichtung eine UV Lampeneinheit 41 (vgl. Fig. 1) vorgesehen sein, so dass UV Licht von dieser UV Lampeneinheit 41 auf die Region abgestrahlt wird, wo die Beprobung der Eier stattfindet.

Insbesondere kann beispielsweise eine UV Röhre verwendet werden, die sich entlang der z-Achse erstreckt, d.h. entlang der Richtung der nebeneinander liegenden Eier. Insbesondere hat sich herausgestellt, dass eine Platzierung der UV Lampeneinheit 41 zwischen den Probenahmeeinheit 3 und der Probeabgabe- und Reinigungsbaugruppe 26 vorteilhaft ist.

Zudem kann die Schrägstellung, mit der die Zuführeinrichtung 1 die Horde zuführt, auch selektiv in Abhängigkeit von Eiercharakteristika geändert werden. Die Einstellungen der gesamten Vorrichtung können über die Kontrolleinheit 6 stattfinden.

Insbesondere, soweit die Eieruntersuchungseinrichtung mit einem System zur Lagepositionsbestimmung der Eier, beispielsweise einem optischen Kamerasystem ausgestattet ist, können diese Bildinformationen verarbeitet werden, um selektiv in Abhängigkeit der Eierdicke, der Eiergröße, die Positionierung der einzelnen Eier zum Entnehmen der Flüssigkeitsmenge festzulegen, und/oder die Kanülenlänge festzulegen.

Für eine selektive auf die Eibeschaffenheit abgestimmte Entnahme von Flüssigkeitsmengen können die Vakuumschläuche derart ausgebildet sein, dass deren Druck jeweils variabel unabhängig voneinander eingestellt werden kann.

Zudem kann es anstelle, dass die einzelnen Probeentnahmeeinrichtungen an einer Grundplatte befestigt sind auch vorteilhaft sein, dass die einzelnen Probeentnahmeeinrichtungen relativ zueinander bewegt werden können.

Soweit bei der Erfindung auf die Aushebeeinrichtung abgestellt wird, kann diese auch gänzlich weggelassen werden und jede Kombination bzw. Ausgestaltung von Probeentnahmeeinrichtung, Spüleinrichtung, Probenaufnahmeeinrichtung, Horde, Kontrolleinrichtung, Positionsbestimmungseinrichtung, oder auch ein einzelnes dieser Elemente für sich eine Erfindung bilden. Die zuvor genannten Einrichtungen bzw. Elemente können jeweils auch unabhängig von den anderen Bestandteilen der Gesamteinrichtung eine eigene Erfindung bilden.

Die Offenbarung der deutschen Patentanmeldung Nr. 10 2015 226 490.4 zum Verfahren und der Details zur Bestimmung der Östronsulfatkonzentration in Allantoisflüssigkeit mittels der Doppelantikörpertechnik wird mittels dieses Verweises zum Offenbarungsgehalt der vorliegenden Anmeldung gemacht wird.

### Bezugszeichenliste

Zuführeinrichtung 1
Aushebeeinrichtung 2
Probeentnahmeeinrichtung 3
Probeaufnahmeeinrichtung 4
Spüleinrichtung 5
Kontrolleinheit 6
Eierstempel 7
Anschlagelement 8
Spritze 9
Grundplatte 10
Vakuumschlauch 11
Kanüle 12
Horde 13
Rahmen 14
Schiene 16
Gitter 17
Ei 18
Anschlagsfläche 19
Kanüle 12
Befestigungselement 20
Plattenelement 21, 22
Lichtschranke 25
Probeabgabe und Reinigungsbaugruppe 26
Wanne für Alkohol 28
Auffanggefäß 29
Computerbildschirm 30
Lehre 31
Stufe 32
Gegenfläche 33
Abdeckung 35
Erste Abstellfläche 36
Zweite Abstellfläche 37
Element aus weichem Material 38
Konischer Kunststoffring 39
UV Lampeneinheit 41
Erster Aktuator A1
Zweiter Aktuator A2
Dritter Aktuator A3
Vierter Aktuator A4
Fünfter Aktuator A5
Sechster Aktuator A6
Siebter Aktuator A7

## Patentansprüche

1. Eine Eieruntersuchungseinrichtung mit,
einer Probeentnahmeeinrichtung (3) mittels welcher einem jeweiligen Ei (18) einer mit Eiern bestückten Horde (13) eine zu entnehmende Flüssigkeitsprobe entnehmbar ist,
einer Zuführeinrichtung (1) zur Zuführung der mit Eiern bestückten Horde (13) zu der Probeentnahmeeinrichtung (3),
und einer Kontrolleinheit (6), mittels welcher die Zuführeinrichtung (1) und die Probeentnahmeeinrichtung (3) steuerbar ist,
**dadurch gekennzeichnet, dass**
die Zuführeinrichtung (1) derart ausgebildet ist, dass diese die Horde (13) unter einem schrägen Winkel von zwischen 20° und 80° zu einer Ebene senkrecht zur Richtung der Schwerkraft zu der Probeentnahmeeinrichtung (3) zuführt, sodass das jeweilige Ei (18) in eine Probenahmeposition verbracht wird, in welcher das Ei (18) von der Probenentnahmeeinrichtung schräg zu seiner Rotationsache anstechbar ist, und das dem Ei (18) in der Probenahmeposition mittels der Probeentnahmeeinrichtung (3) die zu entnehmende Flüssigkeitsmenge entnehmbar ist.

2. Die Eieruntersuchungseinrichtung nach Anspruch 1, **dadurch gekennzeichnet, dass** die Probeentnahmeeinrichtung (3) eine Kanüle (12) aufweist, welche mit einer Vakuumerzeugungseinrichtung verbunden ist, wobei die aus dem Ei (18) zu entnehmende Flüssigkeitsmenge mittels der Kontrolleinheit (6) über den in der Vakuumerzeugungseinrichtung erzeugten Druck steuerbar ist.

3. Die Eieruntersuchungseinrichtung nach einem der vorherigen Ansprüche, **dadurch gekennzeichnet, dass** die Probeentnahmeeinrichtung (3) in Schwerkraftrichtung bewegbar ist; und parallel zu der Fläche innerhalb der die Horde (13) durch die Zuführeinrichtung translatorisch bewegbar ist, und in Richtung quer zur durch die Zuführeinrichtung vorgeschriebene Translationsrichtung der Horde (13) bewegbar ist.

4. Die Eieruntersuchungseinrichtung nach einem der vorherigen Ansprüche, **dadurch gekennzeichnet, dass** die Eieruntersuchungseinrichtung des Weiteren eine Probenaufnahmeeinrichtung (4) aufweist, welche die von der Probeentnahmeeinrichtung (3) aus dem Ei (18) entnommene aus der Probeentnahmeeinrichtung (3) abgegeben Flüssigkeitsmenge aufnimmt.

5. Die Eieruntersuchungseinrichtung nach Anspruch 4 **dadurch gekennzeichnet, dass** die Eieruntersuchungseinrichtung des Weiteren zumindest zwei in einer Einheit zusammengefasste Probenaufnahmeeinrichtungen (4) aufweist, aus welcher die jeweilige von den zumindest zwei Probeentnahmeeinrichtungen (3) aus dem jeweiligen Ei (18) entnommene Flüssigkeitsmenge abgegeben werden kann.

6. Die Eieruntersuchungseinrichtung nach Anspruch 5, **dadurch gekennzeichnet, dass** die zumindest zwei in der Einheit zusammengefassten Probenaufnahmeeinrichtungen (4) durch einen Vlies oder eine Titerplatte ausgebildet sind.

7. Die Eieruntersuchungseinrichtung nach einem der vorherigen Ansprüche, **dadurch gekennzeichnet, dass** die Eieruntersuchungseinrichtung des Weiteren eine eine Positionierbestimmungseinrichtung aufweist, über welche die Lage des Eies (18) bestimmbar ist, wobei die Kontrolleinheit (6) anhand der Daten der Positionierbestimmungseinrichtung eine Positionierung des Eies (18) steuert.

## Claims

1. Examination device for eggs comprising:
a sampling device (3) by means of which a liquid sample to be taken can be taken from a respective egg (18) of a tray (13) filled with eggs,
a feeding device (1) for feeding the tray (13) filled with eggs to the sampling device (3),
and a control unit (6) by means of which the feeding device (1) and the sampling device (3) can be controlled,
**characterised in that**
the feeding device (1) is designed in such a way that it feeds the tray (13) to the sampling device (3) at an oblique angle of between 20° and 80° to a plane perpendicular to the direction of gravity, so that the respective egg (18) is brought into a sampling position in which the egg (18) can be pierced by the sampling device at an angle to its rotation axis, and so that the amount of liquid to be taken can be taken from the egg (18) in the sampling position by means of the sampling device (3).

2. Examination device for eggs according to claim 1, **characterised in that** the sampling device (3) comprises a cannula (12) which is connected to a vacuum generating device, wherein the amount of liquid to be taken from the egg (18) can be controlled by means of the control unit (6) via the pressure generated in the vacuum generating device.

3. Examination device for eggs according to one of the preceding claims, **characterised in that** the sampling device (3) is movable in the direction of gravity; and is translationally movable parallel to the surface within the tray (13) by the feeding device, and is movable in the direction transverse to the translation direction of the tray (13) dictated by the feeding device.

4. Examination device for eggs according to one of the preceding claims, **characterized in that** the examination device for eggs further comprises a sample receiving device (4) which receives the amount of liquid taken from the egg (18) by the sampling device (3) and dispensed from the sampling device (3).

5. Examination device for eggs according to claim 4, **characterised in that** the examination device for eggs further comprises at least two sample receiving devices (4) combined in one unit, from which the respective amount of liquid taken from the respective egg (18) by the at least two sampling devices (3) can be dispensed.

6. Examination device for eggs according to claim 5, **characterised in that** the at least two sample receiving devices (4) combined in the unit are formed by a nonwoven or a titre plate.

7. Examination device for eggs according to any one of the preceding claims, **characterised in that** the examination device for eggs further comprises a positioning determination device by means of which the position of the egg (18) can be determined, wherein the control unit (6) controls the positioning of the egg (18) on the basis of the data from the positioning determination device.

## Revendications

1. Dispositif d'examen d'œufs comprenant:
un dispositif d'échantillonnage (3), au moyen duquel un échantillon de liquide à prélever peut être prélevé d'un œuf (18) respectif d'un plateau (13) équipé d'œufs,
un dispositif d'alimentation (1) pour alimenter le plateau (13) équipé d'œufs au dispositif d'échantillonnage (3),
et une unité de contrôle (6), au moyen de laquelle le dispositif d'alimentation (1) et le dispositif d'échantillonnage (3) peuvent être contrôlés,
**caractérisé en ce que**
le dispositif d'alimentation (1) est conçu de telle façon, que celui-ci alimente le plateau (13) au dispositif d'échantillonnage (3) sous un angle oblique compris entre 20° et 80° par rapport à un plan perpendiculaire à la direction de la gravité, de sorte que l'œuf (18) respectif est placé dans une position d'échantillonnage, dans laquelle l'œuf (18) peut être percé par le dispositif d'échantillonnage en oblique par rapport à son axe de rotation, et que la quantité de liquide à prélever peut être prélevée de l'œuf (18) dans la position d'échantillonnage au moyen du dispositif d'échantillonnage (3).

2. Dispositif d'examen d'œufs selon la revendication 1, **caractérisé en ce que** le dispositif d'échantillonnage (3) comprend une canule (12) qui est reliée à un dispositif de génération de vide, où la quantité de liquide à prélever de l'œuf (18) peut être contrôlée via l'unité de contrôle (6) au moyen de la pression générée dans le dispositif de génération de vide.

3. Dispositif d'examen d'œufs selon l'une des revendications précédentes, **caractérisé en ce que** le dispositif d'échantillonnage (3) est mobile dans la direction de la gravité ; et est mobile en translation parallèlement à la surface à l'intérieur du plateau (13) par le dispositif d'alimentation, et est mobile dans une direction transversale à la direction de la translation du plateau (13) imposée par le dispositif d'alimentation.

4. Dispositif d'examen d'œufs selon l'une des revendications précédentes, **caractérisé en ce que** le dispositif de test des œufs comprend en outre un dispositif de réception d'échantillon (4) qui reçoit la quantité de liquide prélevée de l'œuf (18) par le dispositif d'échantillonnage (3) et déchargée du dispositif d'échantillonnage (3).

5. Dispositif d'examen d'œufs selon la revendication 4, **caractérisé en ce que** le dispositif de test des œufs comprend en outre au moins deux dispositifs de réception d'échantillons (4) réunis dans une unité, à partir de laquelle la quantité respective de liquide prélevée de l'œuf (18) respectif peut être déchargée par les au moins deux dispositifs d'échantillonnage (3).

6. Dispositif d'examen d'œufs selon la revendication 5, **caractérisé en ce que** les au moins deux dispositifs de réception d'échantillons (4) réunis dans l'unité sont formés par un non-tissé ou une plaque de titre.

7. Dispositif d'examen d'œufs selon l'une quelconque des revendications précédentes, **caractérisé en ce que** le dispositif d'examen d'œufs comprend en outre un dispositif de détermination de positionnement au moyen duquel la position de l'œuf (18) peut être déterminée, où l'unité de contrôle (6) contrôle le positionnement de l'œuf (18) à l'aide des données du dispositif de détermination de positionnement.
